# EUROPÄISCHE PATENTSCHRIFT

(11) **EP 1 389 188 B1**
(45) Veröffentlichungstag und Bekanntmachung des Hinweises auf die Patenterteilung: **28.06.2006**
(21) Anmeldenummer: 02771650.5
(22) Anmeldetag: 17.05.2002
(51) Int. Cl.: C07D 211/32, C07D 211/20

(54) **VERFAHREN ZUR HERSTELLUNG VON BIPERIDEN II**
METHOD FOR THE PRODUCTION OF BIPERIDEN II
PROCEDE DE FABRICATION DE BIPERIDES II

(30) Priorität: 18.05.2001 DE 10124449
(43) Veröffentlichungstag der Anmeldung: 18.02.2004
(73) Patentinhaber: LABORATORIO FARMACEUTICO S.I.T. SPECIALITA' IGIENICO TERAPEUTICHE S.r.l., 27035 Mede (Pavia) (IT)
(72) Erfinder: KLEIN, Peter, 67134 Birkenheide (DE); THYES, Marco, 67061 Ludwigshafen (DE); GROSSE, Markus, 68723 Schwetzingen (DE); WEBER, Klaus, Martin, 67346 Speyer (DE); VILSMAIER, Elmar, 67731 Otterbach (DE)
(74) Vertreter: De Gregori, Antonella
(86) Internationale Anmeldenummer: PCT/EP2002/005500
(87) Internationale Veröffentlichungsnummer: WO 2002/094781

(56) Entgegenhaltungen:
- DE-B- 1 005 067
- US-A- 2 789 110
- ELTZE, MANFR. ET AL: "Affinity and selectivity of biperiden enantiomers for muscarinic receptor subtypes" EUROPEAN JURNAL OF PHARMACOLOGY, [Online] Bd. 158, - 1988 Seiten 11-19, XP002206676 Elsevier science[gefunden am 2002-07-15]

## Beschreibung

Die vorliegende Erfindung betrifft ein Verfahren zur Herstellung von Biperiden (Ia).

Biperiden (Ia) ist ein bekanntes zentrales Anticholinergikum und wird zur Behandlung der Parkinsonschen Krankheit eingesetzt (Ullmanns Enzyklopädie der technischen Chemie, 4. Aufl., Band 21, Verlag Chemie, 1982, S. 627). Es handelt sich um ein Racemat aus 1-(Bicyclo[2.2.1]hept-5-en-2-yl(exo,R))-1-phenyl-3-piperidino-propanol(1,S) und 1-(Bicyclo[2.2.1]hept-5-en-2-yl(exo,S))-1-phenyl-3-piperidinopropanol(1,R) (Ia) und stellt eines von vier möglichen Enantiomerenpaaren (Ia-d) des Aminoalkohols 1-(Bicyclo-[2.2.1]hept=5-en-2-yl)-1-phenyl-3-piperidino-1-propanol (I) dar.

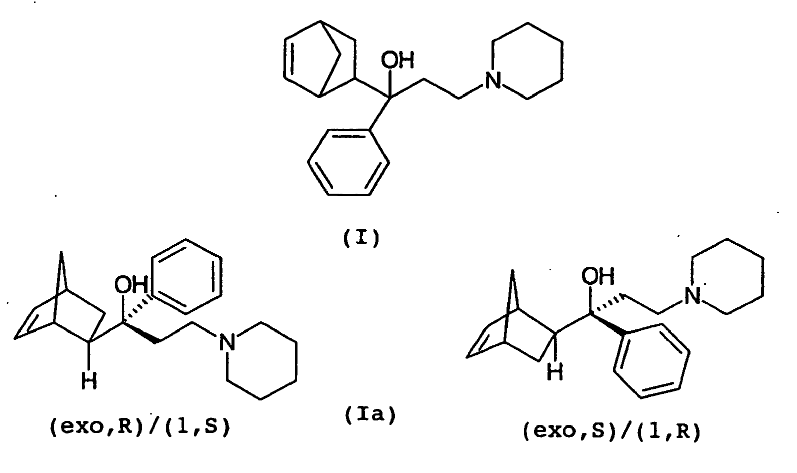

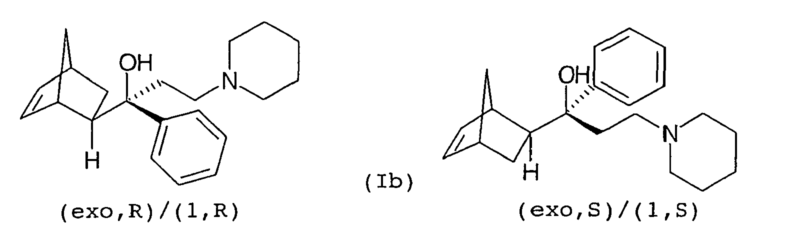

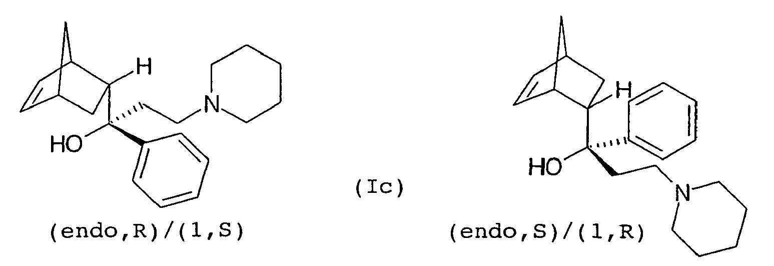

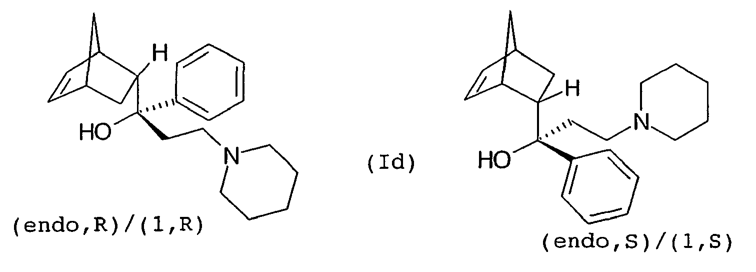

Die DE 1 005 067 und die US 2,789,110 beschreiben die Herstellung des Aminoalkohols I durch Umsetzung von 1-(Bicyclo[2.2.1]hept-5-en-2-yl)-3-piperidino-1-propanon (II) mit einem Phenylmagnesiumhalogenid. Die US 2,789,110 beschreibt zudem die Herstellung des Propanons II, ausgehend von 1-(Bicyclo[2.2.1]hept-5-en-2-yl)-ethanon (III), Paraformaldehyd und Piperidinhydrochlorid in einer Reaktion nach Mannich sowie die Herstellung des Ethanons III aus Cyclopentadien und Methylvinylketon in einer Cycloaddition nach Diels und Alder.

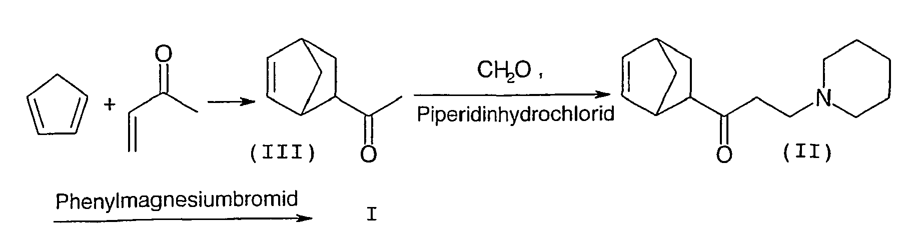

Weder die DE 1 005 067 noch die US 2,789,110 offenbaren, ob es sich bei dem so erhaltenen Aminoalkohol I um ein Isomerengemisch oder um ein reines Isomer handelt.

Das Edukt der Propanolherstellung, 1-(Bicyclo[2.2.1]hept-5-en-2-yl)-3-piperidino-1-propanon (II), kann in zwei isomeren Formen, als exo- oder als endo-Isomeres (II-exo, II-endo), vorliegen, wobei nur die exo-Form in der oben genannten Reaktion mit einem Phenylmagnesiumhalogenid Biperiden (Ia) ergeben kann.

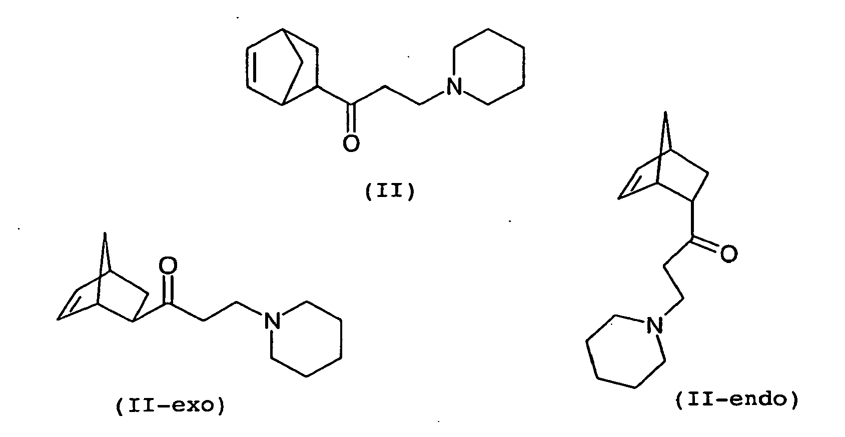

Die Strukturformeln des II-exo und des II-endo zeigen der Einfachheit halber jeweils nur eines von zwei möglichen Enantiomeren des exo- bzw. endo-Isomers. Im Folgenden bezieht sich die Bezeichnung II-exo bzw. II-endo jedoch auf das Enantiomerenpaar der exo- bzw. endo-Form.

Auch 1-(Bicyclo[2.2.1]hept-5-en-2-yl)ethanon (III), die Ausgangssubstanz für die Synthese des Propanons II, kann sowohl als exoals auch als endo-Isomeres vorliegen (III-exo, III-endo) und entsprechend führt nur die Umsetzung des exo-Isomeren in den Folgeschritten zu Biperiden (Ia).

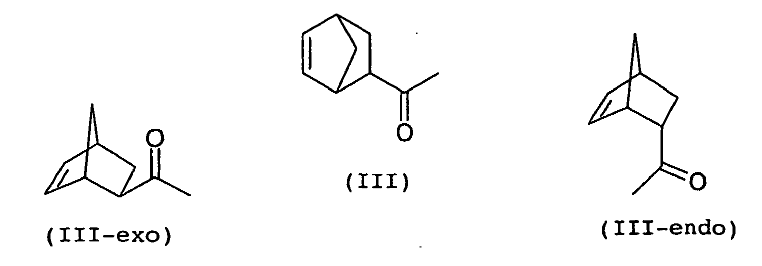

Die Strukturformeln des III-exo und des III-endo zeigen der Einfachheit halber jeweils nur eines von zwei möglichen Enantiomeren des exo- bzw. endo-Isomers. Im Folgenden bezieht sich die Bezeichnung III-exo bzw. III-endo jedoch auf das Enantiomerenpaar der exo- bzw. endo-Form.

Keiner der oben genannten Schriften lassen sich Angaben zur Konfiguration der eingesetzten Edukte III und zwischenprodukte II entnehmen.

Es ist bekannt, dass das 1-(Bicyclo[2.2.1]hept-5-en-2-yl)ethanon (III) aus der Cycloaddition in einem exo/endo-Verhältnis von 1:4 erhalten wird (z.B. R. Breslow, U. Maitra, Tetrahedron Letters, 1984, 25, 1239). Da der eingangs genannte Stand der Technik keinerlei Angaben zur Stereochemie des Ethanons III macht, ist davon auszugehen, dass das Ethanon III in diesem Isomerenverhältnis zur Herstellung des Aminoalkohols I eingesetzt wurde.

Die Herstellung von exo-1-(Bicyclo[2.2.1]hept-5-en-2-yl)ethanon (III-exo) ist 1965 von J.G. Dinwiddie und S.P. McManus beschrieben worden (J. Org. Chem., 1965, 30, 766). Dabei werden exo/endo-Gemische von 1-(Bicyclo[2.2.1]hept-5-en-2-yl)ethanon (III), in denen der endo-Anteil überwiegt, in Methanol in Gegenwart von Natriummethanolat erhitzt und isomerisieren zu Gemischen mit einem exo-Anteil von ca. 70 %. Aus diesen kann durch fraktionierte Destillation und gegebenenfalls Redestillieren des Destillats exo-1-(Bicyclo[2.2.1]hept-5-en-2-yl)ethanon (III-exo) mit einem Reinheitsgrad von bis zu 95 % erhalten werden.

Versuche der Anmelderin haben gezeigt, dass man auch bei der Verwendung von nahezu einheitlichem exo-Ethanon III-exo, d. h. von einem Ethanon III mit einem exo-Anteil von wenigstens 95 %, als Ausgangsmaterial der Mannich-Reaktion stets ein exo/endo-Gemisch des Propanons II erhält, das ein maximales exo/endo-Verhältnis von 4,0:1 aufweist. Dies ist im Hinblick auf die Gewinnung von sauberem Biperiden (Ia) aus der Umsetzung des Propanons II mit einer Phenylmagnesiumverbindung unbefriedigend. Unter sauberem Biperiden versteht man ein Biperiden (Ia) mit einer Reinheit von wenigstens 99,0 %, wie sie für pharmazeutische Anwendungen in der Regel erforderlich ist.

Der vorliegenden Erfindung liegt die Aufgabe zugrunde, ein Verfahren zur Herstellung von Biperiden (Ia) bereit zu stellen, das dieses in einer höheren Ausbeute liefert. Unter Biperiden (Ia) soll eine Substanz der Strukturformel Ia verstanden werden. Insbesondere gilt es, die Selektivität der Propanon 11-Herstellung hinsichtlich des exo-Isomers zu verbessern.

Die Aufgabe konnte gelöst werden durch ein Verfahren zur Herstellung von Biperiden (Ia) durch Umsetzung eines exo/endo-Gemischs von 1-(Bicyclo[2.2.1]hept-5-en-2-yl)-3-Piperidin-1-propanon (II) mit einem exo/endo-Verhältnis von wenigstens 4,5:1 mit einer Phenylmagnesiumverbindung ausgewaehlt zwischen Diphenylmagnesium oder Phenylmagnesiumverbindung der allgemeinen Formel 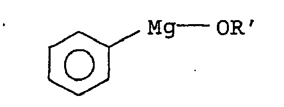 worin R' für C₁-C₄-Alkyl, wie Methyl, Ethyl, n-Propyl, iso-Propyl oder n-Butyl, C₄-C₆-Cycloalkyl, wie Cyclohexyl, C₄-C₆-Cycloalkyl-C₁-C₄-alkyl, wie 2-Cyclohexylethyl, Phenyl-C₁-C₄-alkyl, wie Benzyl, 2-Phenylethyl oder 3-Phenylpropyl, substituiertes Phenyl-C₁-C₄-alkyl, wie 3,4-(Methylendioxy)benzyl, Heteroaryl, wie 8-Chinolyl, Heteroaryl-C₁-C₄-alkyl, wie Furfuryl, 2-Thienylmethyl oder 2-(2-Thienyl)ethyl, oder Benzhydryl steht, 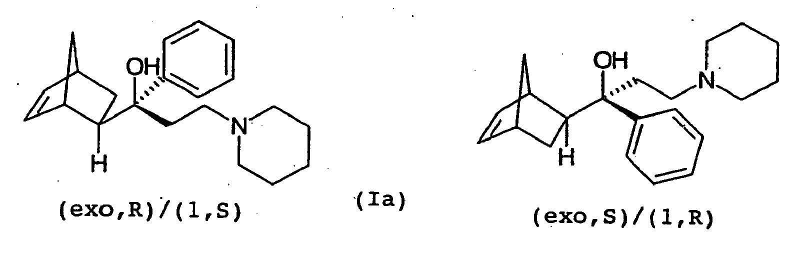 dadurch gekennzeichnet, dass die Herstellung des exo/endo-Gemischs von 1-(Bicyclo[2.2.1]hept-5-en-2-yl)-3-piperidino-1-propanon (II) folgende Schritte umfasst:
a) Umsetzung von exo-1-(Bicyclo[2.2.1]hept-5-en-2-yl)ethanon (III-exo) mit einer Formaldehydquelle und einem Säureadditionssalz von Piperidin oder mit einer Formaldehydquelle und Piperidin in Anwesenheit einer Säure in einem organischen Lösungsmittel oder im Gemisch davon mit Wasser,
b) Überführen des entstandenen Reaktionsgemischs in eine wässrige Lösung und Extraktion dieser wässrigen Lösung mit einem mit Wasser begrenzt oder nicht mischbaren organischen Lösungsmittel bei einem pH-Wert von maximal 7, und
c) Extraktion des in b) erhaltenen Raffinats, welches das exo/endo-Gemisch des 1-(Bicyclo[2.2.1]hept-5-en-2-yl)-3-piperidino-1-propanons (II) enthält, bei einem pH-Wert von wenigstens 7,5 mit einem mit Wasser begrenzt oder nicht mischbaren organischen Lösungsmittel,
d) Abtrennen des organischen Extrakts, Reinigung des organischen Extrakts über einen Auszug mit wässriger Säure und anschließendes Entfernen des Lösungsmittels, wobei man 1-(Bicyclo-[2.2.1] hept-5-en-2-yl)-3-piperidino-1-propanon (II) mit einem exo/endo-verhältnis von wenigstens 4,5:1 erhält.

Unter exo-1-(Bicyclo[2.2.1]hept-5-en-2-yl)ethanon (III-exo) soll im Folgenden ein Ethanon III mit einem exo-Anteil von wenigstens 96 % verstanden werden.

Bei den im erfindungsgemäßen Verfahren eingesetzten exo- und endo-Isomeren handelt es sich, wie bereits für das exo- und endo-Ethanon III-exo und III-endo sowie für das exo- und endo-Propanon II-exo und II-endo beschrieben, um Enantiomerenpaare. Um zu Biperiden (Ia) zu gelangen, das selbst ein Racemat ist, werden racemische Enantiomerengemische der Ausgangsstoffe und der Zwischenprodukte eingesetzt. Das erfindungsgemäße Verfahren kann jedoch auch auf reine Enantiomere sowie auf nicht racemische Enantiomerengemische angewendet werden.

Die Umsetzung im Sinne einer Mannich-Reaktion von exo-1-(Bicyclo-[2.2.1]hept-5-en-2-yl)ethanon (III-exo) mit einer Formaldehydquelle und einem Säureadditionssalz von Piperidin bzw. mit einer Formaldehydquelle und Piperidin in Anwesenheit einer Säure erfolgt im Allgemeinen in einem für Mannich-Reaktionen geeigneten Lösungsmittel. Geeignete Lösungsmittel sind insbesondere C₁-C₄-Alkanole, z. B. Methanol, Ethanol, n-Propanol, Isopropanol, sek-Butanol oder Isobutanol, und ihre Gemische mit Wasser. Vorzugsweise wird Isopropanol verwendet.

Als Säuren kommen grundsätzlich die für eine Aminomethylierung nach Mannich geeigneten Mineralsäuren oder organischen Säuren in Betracht.

Vorzugsweise verwendet man Salzsäure bzw. Chlorwasserstoff oder eine organische Sulfonsäure der allgemeinen Formel RSO₃H. R steht hierbei für einen einwertigen organischen Rest, vorzugsweise für C₁-C₄-Alkyl, Phenyl oder C₁-C₄-Alkyl-substituiertes Phenyl, wobei Methyl besonders bevorzugt ist. Die Umsetzung kann mit dem Säureadditionssalz von Piperidin oder aber mit Piperidin in Anwesenheit einer Säure erfolgen, wobei hier das Säureadditionssalz von Piperidin in situ gebildet wird.

In diesem Fall werden Piperidin und die entsprechende Säure vorzugsweise äquimolar eingesetzt. Im Falle der Verwendung des isolierten Säureadditionssalzes von Piperidin kann dieses, falls nicht kommerziell erwerblich, durch die Umsetzung von Piperidin mit einer entsprechenden Säure in Molverhältnissen im Bereich von 1:0,9 bis 1:2, vorzugsweise im Bereich von 1:0,9 bis 1:1,5, insbesondere im Bereich von 1:0,9 bis 1:1,2 und besonders bevorzugt etwa äquimolar und durch eine anschließende Isolierung hergestellt werden.

Vorzugsweise wird das isolierte Säureadditionssalz von Piperidin in die Mannichreaktion eingesetzt. Besonders bevorzugt wird Piperidinhydrochlorid oder Piperidiniummethansulfonat eingesetzt.

Vorzugsweise setzt man das exo-Ethanon III-exo und die Formaldehydquelle in einem Molverhältnis von 1:1 bis 1:2 ein, wobei man die Formaldehydquelle insbesondere in einem Überschuss von 10 bis 100 Mol-% und besonders bevorzugt von 10 bis 30 Mol-%, z. B. 20 Mol-%, einsetzt. Als Formaldehydquelle eignen sich gasförmiges Formaldehyd, Formalin, Trioxan oder Paraformaldehyd. Bevorzugt verwendet man Paraformaldehyd.

Vorzugsweise setzt man das exo-Ethanon III-exo und Piperidin bzw. das Säureadditionssalz von Piperidin in einem Molverhältnis von 1:0,9 bis 1:2, insbesondere von 1:0,95 bis 1:1,5 und besonders bevorzugt von 1:1 bis 1:1,3 ein.

Bei der Verwendung von Salzsäure bzw. Chlorwasserstoff als Säure werden das exo-Ethanon III-exo und Piperidin bzw. sein Säureadditionssalz in einem Molverhältnis im Bereich von 1:0,9 bis 1:1,5, vorzugsweise im Bereich von 1:0,9 bis 1:1,2 und besonders bevorzugt etwa äquimolar umgesetzt. In einer speziellen Ausführungsform betragen die Molverhältnisse der Komponenten exo-Ethanon III-exo, Piperidin bzw. sein Säureadditionssalz und Formaldehydquelle 1:1-1,01:1,2.

Bei der Verwendung einer Sulfonsäure werden das exo-Ethanon III-exo und Piperidin bzw. sein Säureadditionssalz in der Regel in einem Molverhältnis im Bereich von 1:1 bis 1:2 eingesetzt. Vorzugsweise wird Piperidin bzw. sein Säureadditionssalz im Überschuss eingesetzt, wobei man bevorzugt einen Überschuss von 10 bis 100 Mol-%, besonders bevorzugt von 10 bis 30 Mol-%, z. B. 20 Mol-%, verwendet. Piperidin bzw. sein Säureadditionssalz und die Formaldehydquelle werden dabei geeigneterweise in einem Molverhältnis im Bereich von 1:0,9 bis 1:1,2, vorzugsweise etwa äquimolar eingesetzt. In einer speziellen Ausführungsform betragen die Molverhältnisse der Komponenten exo-Ethanon III-exo, Piperidin bzw. sein Säureadditionssalz und Formaldehydquelle 1:1,2:1,2.

Die Reaktionstemperatur der Mannich-Reaktion liegt in der Regel im Bereich von 0 °C bis zur Siedetemperatur des Reaktionsgemischs. Vorzugsweise erhitzt man zum Rückfluss. Die Reaktionsdauer beträgt gewöhnlich 2 bis 24 Stunden, vorzugsweise 5 bis 12 Stunden und besonders bevorzugt 5 bis 8 Stunden.

Die Überführung des in Schritt a) erhaltenen Reaktionsgemischs erfolgt in der Regel in der Weise, dass man zunächst das organische Lösungsmittel aus dem Reaktionsgemisch entfernt, was üblicherweise destillativ, vorzugsweise unter vermindertem Druck vorgenommen wird. Der Rückstand wird dann, sofern erforderlich, in Wasser aufgenommen, d. h. sofern man einen festen oder öligen Rückstand erhält. Wenn eine wässrige Mischung erhalten wird, kann diese gegebenenfalls mit Wasser verdünnt werden. Die so erhaltenen wässrigen Gemische werden zur Entfernung nicht-basischer organischer Bestandteile - in der Regel nicht umgesetztes Ausgangsmaterial - mit einem mit Wasser begrenzt oder nicht mischbaren organischen Lösungsmittel ein- oder mehrfach extrahiert, wobei der pH-Wert der wässrigen Phase einen Wert von 7,0 nicht überschreitet. Zu den geeigneten, mit Wasser begrenzt oder nicht mischbaren Lösungsmitteln zählen C₅-C₈-Aliphaten wie n-Pentan oder n-Hexan, C₅-C₆-Alicyclen wie Cyclohexan, Aromaten wie Benzol, Toluol oder Xylole und aliphatische C₄-C₈-Ether wie Diethylether, tert-Butylmethylether oder Diisopropylether bzw. Mischungen davon. Vorzugsweise wird mit aliphatischen C₄-C₈-Ethern, insbesondere mit Diisopropylether extrahiert.

Hierzu wird man vorzugsweise so vorgehen, dass man zunächst die in der Regel noch saure wässrige mit dem mit Wasser nicht mischbaren Lösungsmittel extrahiert. Vorzugsweise wird man die wässrige Mischung mehrmals, insbesondere 2 bis 5 mal und speziell 3 mal extrahieren.

Anschließend kann man das Raffinat mit einer Base abstumpfen, wobei der pH-Wert ≤ 7 eingehalten wird, und erneut extrahieren. Diese Vorgehensweise ist bevorzugt. Hierzu wird man vorzugsweise die wässrige Lösung ein- bis mehrfach mit einem Gemisch einer Base bzw. einer wässrigen basischen Lösung und einem oder mehreren der oben genannten, zur Extraktion der wässrigen Phase geeigneten Lösungsmittel, vorzugsweise Diisopropylether, behandeln. Als Basen verwendet man üblicherweise Alkali- oder Erdalkalihydroxide oder Alkalicarbonate. Vorzugsweise wird Natriumhydroxid oder Kaliumhydroxid bzw. ihre wässrigen Lösungen und insbesondere Natriumhydroxid bzw. Natronlauge verwendet. Die Gesamtmenge an eingesetzter Base liegt im Bereich von 5 bis 15 Mol-%, vorzugsweise 8 bis 10 Mol-% in Bezug auf die Menge an eingesetztem exo-Ethanon III-exo. Der pH-Wert der wässrigen Phase soll dabei einen pH-Wert von 7 nicht überschreiten.

Die wässrige Lösung wird dann in Schritt c) des erfindungsgemäßen Verfahrens in einer oder mehreren Stufen mit einer der oben genannten Basen bzw. einer wässrigen basischen Lösung, vorzugsweise Natriumhydroxid bzw. Natronlauge, auf einen pH-Wert von wenigstens 7,5, vorzugsweise im Bereich von 7,5 bis 9, insbesondere im Bereich von 8 bis 8,5 und besonders bevorzugt im Bereich von 8,1 bis 8,3, eingestellt. Bei der stufenweisen Alkalisierung der wässrigen Phase wird nach jeder Zugabe der Base bzw. der wässrigen basischen Lösung die wässrige Lösung geeigneterweise mit einem der oben genannten, zur Extraktion der wässrigen Phase geeigneten organischen Lösungsmittel, vorzugsweise Diisopropylether, extrahiert; bei der einstufigen Zugabe der Base bzw. der wässrigen basischen Lösung erfolgt dies entsprechend im Anschluss daran, wobei hier gegebenenfalls mehrfach, z. B. drei- bis fünffach, mit dem organischen Lösungsmittel extrahiert wird.

Die organischen Extrakte, die das 1-(Bicyclo[2.2.1]hept-5-en-2-yl)-3-piperidino-1-propanon (II) enthalten, werden in der Regel vereinigt und in Schritt d) über einen Auszug mit Säure gereinigt. Hierzu wird man in der Regel das in Schritt c) erhaltene organische Extrakt mit einer wässrigen, insbesondere einer verdünnten wässrigen Säure behandeln. Gegebenenfalls kann sich hiernach ein Waschen mit Wasser anschließen. Als Säure verwendet man in der Regel Mineralsäuren wie Salzsäure, Bromwasserstoffsäure, Schwefelsäure oder Phosphorsäure, bevorzugt wird Salzsäure verwendet. Die Menge an eingesetzter Säure beträgt in der Regel 0,02 bis 0,10 Protonenäquivalente, vorzugsweise 0,03 bis 0,05 Protonenäquivalente, der in der Mannich-Reaktion eingesetzten Menge an exo-Ethanon III-exo. Unter Protonenäquivalenten versteht man die Anzahl der Protonen in einem Säuremolekül. Die Konzentration der wässrigen Säure liegt in der Regel im Bereich von 0,5 bis 10 M und insbesondere im Bereich von 2 bis 7 M.

Anschließend wird der organische Extrakt vom Lösungsmittel befreit, was vorzugsweise im Vakuum erfolgt.

Der nach dem Entfernen des Lösungsmittels verbleibende Eindampfrückstand, der zu wenigstens 95 Gew.-% aus dem exo/endo-Gemisch des Propanons II besteht, enthält dieses in einem exo/endo-Verhältnis von wenigstens 4,5:1, insbesondere von wenigstens 6:1 und im Fall der Verwendung von Piperidiniummethansulfonat als Ausgangsmaterial der Mannich-Reaktion von wenigstens 10:1, insbesondere von wenigstens 15:1, z. B. von 22:1.

Das nach der erfindungsgemäßen Aufarbeitung erhaltene exo/endo-Gemisch des Propanons II enthält bei einer wenigstens gleich großen Gesamtausbeute an exo/endo-Propanon II das exo-Propanon II-exo in einem wesentlich größeren Anteil als das nach der üblichen, destillativen Aufarbeitung erhaltene exo/endo-Gemisch des Propanons II.

Das erfindungsgemäß hergestellte exo/endo-Gemisch von 1-(Bicyclo-[2.2.1]hept-5-en-2-yl)-3-piperidino-1-propanon (II) wird in einer Grignard-Reaktion mit einer Phenylmagnesiumverbindung, vorzugsweise mit Diphenylmagnesium oder besonders bevorzugt mit einer Phenylmagnesiumverbindung der allgemeinen Formel 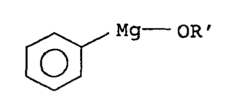 worin R' für C₁-C₄-Alkyl, wie Methyl, Ethyl, n-Propyl, iso-Propyl oder n-Butyl, C₄-C₆-Cycloalkyl, wie Cyclohexyl, C₄-C₆-Cycloalkyl-C₁-C₄-alkyl, wie 2-Cyclohexylethyl, Phenyl-C₁-C₄-alkyl, wie Benzyl, 2-Phenylethyl oder 3-Phenylpropyl, substituiertes Phenyl-C₁-C₄-alkyl, wie 3,4-(Methylendioxy)benzyl, Heteroaryl, wie 8-Chinolyl, Heteroaryl-C₁-C₄-alkyl, wie Furfuryl, 2-Thienylmethyl oder 2-(2-Thienyl)ethyl, oder Benzhydryl steht, in einem geeigneten Lösungsmittel umgesetzt. Die Phenylmagnesiumverbindung der oben dargestellten Formel wird im Folgenden als Phenylmagnesiumalkoxid bezeichnet.

Geeignete Lösungsmittel sind Aromaten wie Benzol, Toluol oder Xylole, acyclische oder cyclische Ether mit 4 bis 6 C-Atomen, Gemische davon oder deren Mischungen mit aliphatischen oder alicyclischen Kohlenwasserstoffen wie n-Hexan bzw. Cyclohexan. Geeignete acyclische Ether sind z. B. Diethylether und tert-Butylmethylether, geeignete cyclische Ether sind z. B. Tetrahydrofuran und Dioxan. Bevorzugt verwendet man Diethylether, Tetrahydrofuran oder Dioxan oder Mischungen davon. Die Lösungsmittel werden in der Regel, wie für Grignard-Reaktionen üblich, wasserfrei eingesetzt.

Das Phenylmagnesiumalkoxid wird in allgemein bekannter Weise, z. B. durch Umsetzung von Diphenylmagnesium mit einem Alkohol der allgemeinen Formel R'OH, worin R' wie oben definiert ist, hergestellt. Diphenylmagnesium und der Alkohol werden dabei in einem Molverhältnis im Bereich von 1:0,9 bis 1:1,5, vorzugsweise im Bereich von 1:1 bis 1:1,2 und besonders bevorzugt etwa äquimolar umgesetzt. Gewöhnlich wird Diphenylmagnesium, das üblicherweise wie weiter unten beschrieben in situ erzeugt wird, in einem der oben genannten, für Grignard-Reaktionen geeigneten Lösungsmittel vorgelegt und der Alkohol in der Regel portionsweise über eine Zeitspanne von 5 Minuten bis etwa eine Stunde bei einer Temperatur von 0 bis 80 °C, vorzugsweise von 0 bis 50 °C und besonders bevorzugt von 0 bis 40 °C zugegeben. Nach beendeter Zugabe kann man das Gemisch im selben Temperaturbereich zur Vervollständigung der Reaktion noch 15 Minuten bis 2 Stunden, vorzugsweise 15 Minuten bis eine Stunde, belassen oder vorzugsweise rühren.

Die Herstellung des in dem erfindungsgemäßen Verfahren eingesetzten Diphenylmagnesiums erfolgt in an sich bekannter Weise. Beispielsweise kann man ein Phenylmagnesiumhalogenid, z. B. Phenylmagnesiumchlorid, in einem geeigneten Lösungsmittel mit Dioxan versetzen, wobei unter Verschiebung des Schlenk-Gleichgewichts Diphenylmagnesium und der entsprechende Magnesiumhalogenid-Dioxan-Komplex entstehen. Letzterer fällt in der Regel aus, wird aber bevorzugt nicht aus der Lösung entfernt. Geeignete Lösungsmittel sind im Allgemeinen acyclische und cyclische Ether mit vorzugsweise 4 bis 6 C-Atomen oder deren Mischungen mit aliphatischen, alicyclischen oder aromatischen Kohlenwasserstoffen. Geeignete acyclische Ether sind z. B. Diethylether und tert-Butylmethylether, ein geeigneter cyclischer Ether ist Tetrahydrofuran. Zu den geeigneten aliphatischen bzw. alicyclischen Kohlenwasserstoffen zählen insbesondere n-Hexan bzw. Cyclohexan, geeignete aromatische Kohlenwasserstoffe sind z. B. Benzol, Toluol und Xylole.

Dioxan wird in der Regel mindestens äquimolar im Verhältnis zum Phenylmagnesiumhalogenid eingesetzt. Soll als Phenylmagnesiumverbindung Diphenylmagnesium verwendet werden, so setzt man vorzugsweise Dioxan im Überschuss, beispielsweise in einem Überschuss von 50 bis 500 Mol-%, insbesondere von 100 bis 300 Mol-% und speziell von 100 bis 200 Mol-%, ein. Soll Diphenylmagnesium zuerst in das Phenylmagnesiumalkoxid überführt werden, setzt man vorzugsweise Dioxan und das Phenylmagnesiumhalogenid in einem Molverhältnis im Bereich von 1:1 bis 1,5:1, insbesondere 1:1 bis 1,2:1 und besonders bevorzugt etwa äquimolar ein.

Die Zugabe des Dioxans zu der Lösung des Phenylmagnesiumhalogenids erfolgt in der Regel bei einer Temperatur im Bereich von -20 bis 60 °c, vorzugsweise im Bereich von -10 bis 40 °C.

Üblicherweise belässt man die nach Zugabe des Dioxans erhaltene Mischung noch 15 Minuten bis 2 Stunden, vorzugsweise 20 Minuten bis eine Stunde, in dem für die Zugabe des Dioxans genannten Temperaturbereich, bevor man sie ins erfindungsgemäße Verfahren einsetzt.

Sowohl die Herstellung von Diphenylmagnesium, die Umsetzung zum Phenylmagnesiumalkoxid als auch die Umsetzung der Phenylmagnesiumverbindung mit dem Propanon II nach Grignard erfolgen geeigneterweise unter einer Inertgasatmosphäre. Als Inertgase kommen beispielsweise Stickstoff und die Edelgase wie Argon sowie deren Mischungen in Betracht.

In der Grignard-Reaktion des Propanons II mit der Phenylmagnesiumverbindung setzt man in der Regel die Phenylmagnesiumverbindung und das Propanon II in einem Molverhältnis im Bereich von 0,8:1 bis 3:1, vorzugsweise im Bereich von 0,8:1 bis 2:1 und insbesondere im Bereich von 0,8:1 bis 1,5:1, ein. Besonders bevorzugt wird das Propanon II mit Diphenylmagnesium bzw. mit dem Phenylmagnesiumalkoxid in einem Molverhältnis im Bereich von 1:1 bis 1:1,3 umgesetzt.

In der Regel legt man die Phenylmagnesiumverbindung in Form einer Lösung in einem der oben genannten, für Grignard-Reaktionen geeigneten, organischen Lösungsmittel vor und fügt das Propanon II bei einer Temperatur im Bereich von -20 °C bis zur Siedetemperatur des Reaktionsgemischs, vorzugsweise im Bereich von -10 °C bis 90 °C und besonders bevorzugt im Bereich von 0 °C bis 70 °C hinzu. Dabei wird die Phenylmagnesiumverbindung in der Regel in einer Konzentration im Bereich von 0,1 bis 10 mol/1, vorzugsweise im Bereich von 0,1 bis 3 mol/l und besonders bevorzugt im Bereich von 0,2 bis 2 mol/l eingesetzt.

Die Zugabe des Propanons II kann in einer Portion oder vorzugsweise über einen Zeitraum von wenigen Minuten bis zu mehreren Stunden, z. B. 5 Minuten bis 5 Stunden, erfolgen. Die Zugabe des Propanons II erfolgt entweder in Form einer Lösung in einem der oben genannten, für Grignard-Reaktionen geeigneten, inerten Lösungsmittel oder vorzugsweise in Reinform. Bei der Zugabe als Lösung beträgt die Konzentration des Propanons II in der Regel 0,1 bis 20 mol/1, vorzugsweise 1 bis 15 mol/l. Zur Vervollständigung der Reaktion wird das Reaktionsgemisch üblicherweise 15 Minuten bis 5 Stunden, speziell 30 Minuten bis 2 Stunden bei einer Temperatur im Bereich von -20 °C bis zur Siedetemperatur des Reaktionsgemischs, vorzugsweise im Bereich von -10 °C bis 90 °C und besonders bevorzugt im Bereich von 10 °C bis 80 °C belassen, wobei man vorzugsweise zur besseren Durchmischung rührt. Die Aufarbeitung erfolgt wie für Grignard-Reaktionen üblich wässrig-extraktiv, z. B. indem man das Reaktionsgemisch mit Wasser, einer wässrigen Ammoniumchloridlösung oder einer sauren wässrigen Lösung quencht, wobei man im letzten Fall den pH-Wert des entstandenen Gemischs anschließend alkalisch einstellt, das gequenchte Gemisch gegebenenfalls nach Abtrennung einer organischen Phase mit einem mit Wasser nicht mischbaren, zum Lösen des Produkts geeigneten Lösungsmittel extrahiert und aus dem Extrakt bzw. aus dem mit der organischen Phase vereinigten Extrakt das Lösungsmittel entfernt. Geeignete Lösungsmittel sind beispielsweise Aromaten wie Benzol oder Toluol, die oben genannten acyclischen Ether, Ester wie Ethylacetat oder chlorhaltige Aliphaten wie Dichlor- oder Trichlormethan.

Das aus der Umsetzung des Propanons II mit Diphenylmagnesium bzw. mit dem Phenylmagnesiumalkoxid erhaltene Rohprodukt besteht im Wesentlichen aus den vier diastereomeren Enantiomerenpaaren Ia bis Id des 1-(Bicyclo[2.2.1]hept-5-en-2-yl)-1-phenyl-3-piperidino-1-propanols (I), wobei das Enantiomerenpaar Ia (Biperiden) die Hauptmenge bildet. Das gaschromatographisch bestimmte Verhältnis von Biperiden (Ia) zu den übrigen drei Enantiomerenpaaren Ib bis Id beträgt üblicherweise wenigstens 1,5:1 und liegt vorzugsweise im Bereich von 1,7:1 bis 2,4:1. Besonders hohe Anteile am Enantiomerenpaar Ia erhält man z. B. bei der Umsetzung von Diphenylmagnesium oder Phenylmagnesiumbenzylalkoholat mit dem Propanon II, das aus der Umsetzung des exo-Ethanons III-exo mit Piperidiniummethansulfonat gewonnen wurde.

Zur Isolierung des Biperidens (Ia) aus dem Diastereomerengemisch wird dieses unter Erwärmen, vorzugsweise bei einer Temperatur von 40 bis 80 °C, insbesondere von 50 bis 70 °C, in einem Gemisch aus Wasser und einem polaren, mit Wasser mischbaren organischen Lösungsmittel gelöst. Geeignete Lösungsmittel sind C₁-C₃-Alkanole, d. h. Methanol, Ethanol, n-Propanol und Isopropanol. Vorzugsweise verwendet man wässriges Isopropanol, besonders bevorzugt 70 bis 95%iges Isopropanol und insbesondere 90%iges Isopropanol. Die hier und im Folgenden gemachten %-Angaben bezüglich des Isopropanolgehalts beziehen sich auf das Volumen des Isopropanols bezogen auf das Gesamtvolumen des wasserhaltigen Lösungsmittels. Zu dieser Lösung wird HC1, beispielsweise in Form einer Lösung von Chlorwasserstoff in einem organischen Lösungsmittel, vorzugsweise in einem der genannten C₁-C₃-Alkanole, bevorzugt in Isopropanol, oder in Form von Salzsäure, zugegeben. HCl wird wenigstens äquimolar bezogen auf den Aminoalkohol I, vorzugsweise in einem Überschuss von 5 bis 50 Mol-% und besonders bevorzugt von 5 bis 20 Mol-%, eingesetzt. Die Zugabe erfolgt vorzugsweise bei erhöhter Temperatur, z. B. bei 40 bis 80 °C und insbesondere bei 50 bis 70 °C. Zur Vervollständigung der Reaktion wird nach vollendeter Zugabe das Reaktionsgemisch bei einer Temperatur von 50 °C bis zur Siedetemperatur des Reaktionsgemischs 0,5 bis 3 Stunden belassen, wobei man vorzugsweise rührt. In einer bevorzugten Ausführungsform wird das Reaktionsgemisch zuerst zwei Drittel der Zeit bei 55 bis 65 °C und anschließend ein Drittel der Zeit bei Rückflusstemperatur gerührt. Das Reaktionsgemisch wird anschließend auf eine Temperatur im Bereich von 0 bis 30 °C abgekühlt, gegebenenfalls noch bis zu mehreren Stunden, z. B. bis zu 10 Stunden, vorzugsweise bis zu 5 Stunden, in diesem Temperaturbereich gerührt und dann das gebildete Hydrochlorid in üblicher Weise von der Lösung abgetrennt.

Zur weiteren Aufreinigung des Hydrochlorids wird dieses im Allgemeinen feucht oder trocken in Wasser und einer ausreichenden Menge eines oder mehrerer polarer, mit Wasser begrenzt oder nicht mischbarer Dialkylether mit 4 bis 8 C-Atomen wie Diethylether, tert-Butylmethylether und insbesondere Diisopropylether aufgenommen und das Gemisch mit einer geeigneten Base versetzt. Ausreichende Mengen an organischen Lösungsmitteln sind z. B. 4 bis 10 ml Lösungsmittel pro Gramm trockenen Hydrochlorids. Wasser und organisches Lösungsmittel werden vorzugsweise in einem Volumen-verhältnis im Bereich von 1:2 bis 1:5 eingesetzt.

Geeignete Basen sind Alkali- und Erdalkalihydroxide sowie Alkalicarbonate; besonders bevorzugt werden Natrium- oder Kaliumhydroxid bzw. ihre wässrigen Lösungen und insbesondere Natriumhydroxid bzw. Natronlauge verwendet. Möglich ist aber auch die Verwendung wasserlöslicher organischer Basen, beispielsweise aliphatisch substituierte Amine mit 2 bis 8 C-Atomen. Die Base wird wenigstens äquimolar, vorzugsweise im Überschuss, insbesondere in einem Überschuss von 5 bis 15 Mol-%, bezogen auf das Hydrochlorid, eingesetzt.

Vorzugsweise erfolgt die Umsetzung mit der Base in der Wärme. Hierzu wird vor, während oder bevorzugt nach Zugabe der Base die Mischung auf eine Temperatur im Bereich oberhalb 25 °C bis zur Siedetemperatur des Reaktionsgemischs, vorzugsweise im Bereich von 30 bis 70 °C, bei Verwendung von Diisopropylether als Dialkylether vorzugsweise im Bereich von 40 bis 65 °C, insbesondere von 55 bis 60 °C, erwärmt. Hierbei entstehen in der Regel zwei klare Phasen, die warm, im Falle der Verwendung von Diisopropylether als Dialkylether im oben erwähnten Temperaturbereich, getrennt werden. Die organische Phase wird warm, im Falle der Verwendung von Diisopropylether als Dialkylether im oben erwähnten Temperaturbereich, mit Wasser gewaschen und dann vorzugsweise bei Normaldruck durch Entfernen des Lösungsmittels bis zu einem Gewicht/Volumen-Verhältnis des Produkts zum Lösungsmittel im Bereich von 1:2 bis 1:6, vorzugsweise von 1:3 bis 1:4,5, aufkonzentriert. Beim Abkühlen des Gemischs auf Raumtemperatur oder darunter, vorzugsweise jedoch nicht unter -10 °C, kristallisiert sauberes Biperiden (Ia) aus, das nach üblichen Methoden der Feststoffisolierung, z. B. Abfiltrieren des Feststoffs oder Dekantieren der Mutterlauge, gewonnen wird.

Durch die erfindungsgemäße Verwendung des Propanons II mit einem höheren exo-Anteil konnte die Ausbeute an Biperiden (Ia) erheblich gesteigert werden, insbesondere in Kombination mit der oben beschriebenen Aufarbeitung.

Biperiden (Ia) kann anschließend mit einer pharmakologisch verträglichen Säure in üblicher Weise in sein Säureadditionssalz überführt werden. Geeignete Säuren sind beispielsweise Halogenwasserstoffsäuren, insbesondere Chlorwasserstoff bzw. Salzsäure, sowie organische Mono- oder Dicarbonsäuren wie Essigsäure, Oxalsäure, Maleinsäure, Fumarsäure, Milchsäure, Weinsäure, Adipinsäure oder Benzoesäure, weiterhin Phosphorsäure und Schwefelsäure sowie die in "Fortschritte der Arzneimittelforschung, Band 10, S. 224ff, Birkhäuser Verlag, Basel, Stuttgart, 1966" genannten Säuren. Üblicherweise kommt Biperiden (Ia) als Hydrochlorid in den Handel.

Das zur Herstellung des 1-(Bicyclo[2.2.1]hept-5-en-2-yl)-3-piperidino-1-propanons (II) verwendete exo-1-(Bicyclo[2.2.1]hept-5-en-2-yl)ethanon (III-exo) erhält man durch die Umsetzung von Cyclopentadien und Methylvinylketon in einer Cycloaddition nach Diels und Alder. Eine besonders vorteilhafte Methode zur Darstellung von III mit hohem Anteil an III-exo ist in der deutschen Patentanmeldung P 10124452.5 beschrieben auf deren Offenbarung wegen weiterer Details in vollem Umfang Bezug genommen wird.

Die Cycloaddition von Cyclopentadien und Methylvinylketon kann grundsätzlich in einem für solche Reaktionen gängigen Lösungsmittel wie Diethylether, Benzol, Toluol oder Xylol oder auch ohne Lösungsmittel durchgeführt werden. Vorzugsweise wird kein Lösungsmittel verwendet. Cyclopentadien und Methylvinylketon werden in der Regel in einem Molverhältnis im Bereich von 3,0:1 bis 0,5:1 eingesetzt. Vorzugsweise werden sie äquimolar oder mit Cyclopentadien im Überschuss umgesetzt, wobei der Überschuss vorzugsweise 50 bis 150 Mol-% beträgt.

Die Reaktion wird in der Regel bei einer Temperatur im Bereich von 0 bis 60 °C, vorzugsweise im Bereich von 10 bis 40 °C durchgeführt.

Niedrig siedende Bestandteile, meist unumgesetzte Edukte, werden in der Regel im Anschluss an die Cycloaddition bei erniedrigtem Druck, vorzugsweise bei 1 bis 150 mbar, destillativ entfernt. Das zurückbleibende Gemisch, das zu etwa 20 % aus exo- und zu etwa 80 % aus endo-1-(Bicyclo[2.2.1]hept-5-en-2-yl)ethanon besteht, wird mit einem Alkali-C₁-C₄-alkoholat umgesetzt. Die Menge an Alkalialkoholat beträgt in der Regel 0,1 bis 5 Gew.-%, vorzugsweise 0,2 bis 2 Gew.-%, bezogen auf das Gesamtgewicht der Mischung. Vorzugsweise wird Natriummethanolat verwendet. Die zur Isomerisierung des Ethanons III erforderliche Temperatur liegt in der Regel im Bereich von 50 bis 110 °C, vorzugsweise im Bereich von 60 bis 100 °C. Hierzu erhitzt man häufig die Mischung unter vermindertem Druck zum Rückfluss, vorzugsweise bei einem Druck von 1 bis 100 mbar und insbesondere bei einem Druck von 5 bis 50 mbar. Man wendet diese Bedingungen in der Regel 10 Minuten bis 5 Stunden, insbesondere 20 Minuten bis 3 Stunden und speziell 0,5 Stunden bis 2 Stunden an und beginnt dann das erhaltene Gemisch fraktioniert zu destillieren, wobei bevorzugt das exo-Isomer von III abdestilliert. Man geht davon aus, dass durch das Entfernen des exo-Isomeren aus dem Gleichgewicht die Isomerisierung des endo-Ethanons zur exo-Form gefördert wird. Die fraktionierte Destillation erfolgt in der Regel über eine Kolonne unter vermindertem Druck, vorzugsweise im Bereich von 1 bis 100 mbar, insbesondere von 1 bis 50 und speziell von 1 bis 20 mbar. Die Destillationstemperatur (Kopftemperatur) wird vorzugsweise auf 50 bis 100 °C und speziell auf 50 bis 80 °C eingestellt. Auf diese Weise erhält man exo-1-(Bicyclo[2.2.1]hept-5-en-2-yl)ethanon (III-exo) in einem Reinheitsgrad, der wenigstens 96 % beträgt. Durch Redestillation des Destillats erhält man das exo-Ethanon III-exo in einem Reinheitsgrad von bis zu 100 %.

Die folgenden Beispiele dienen der Veranschaulichung der Erfindung, sind jedoch nicht einschränkend zu verstehen.

### Beispiel

### 1. Herstellung des Ausgangsmaterials

### 1.1 exo-1-(Bicyclo[2.2.1]hept-5-en-2-yl)ethanon (III-exo)

Zu 210,3 g Methylvinylketon wurden zügig 198,3 g Cyclopentadien gegeben. Nach beendeter Zugabe wurde die Reaktionslösung noch eine Stunde bei Raumtemperatur nachgerührt und dann bei einer Temperatur von 58 °C und einem Druck von 20 mbar nicht umgesetztes Edukt destillativ entfernt. Der Eindampfrückstand, hauptsächlich bestehend aus einem Gemisch aus der exo- und der endo-Form von 1-(Bicyclo[2.2.1]hept-5-en-2-yl)ethanon (III) im Verhältnis von 1:4, wurde mit 5 g Natriummethanolat versetzt und eine Stunde bei einem Druck von 10 bis 20 mbar zum Rückfluss erhitzt. Das Reaktionsgemisch wurde anschließend über eine Kolonne bei einer Temperatur von 75 °C und einem Druck von 20 mbar destilliert. Erhalten wurden dabei 298,3 g (73 % der Theorie) exo-1-(Bicyclo-[2.2.1]hept-5-en-2-yl)ethanon (III-exo) in Form eines leicht gelblichen Öls.

### 1.2 1-(Bicyclo[2.2.1]hept-5-en-2-yl)-3-piperidino-1-propanon (II)

### 1.2.1 Herstellung von 1-(Bicyclo[2.2.1]hept-5-en-2-yl)-3-piperidino-1-propanon (II) unter Verwendung von Piperidin-Hydrochlorid

510,7 g exo-1-(Bicyclo[2.2.1]hept-5-en-2-yl)ethanon (III-exo), 460,6 g Piperidin-Hydrochlorid und 135,0 g Paraformaldehyd wurden in 950 ml Isopropanol 7 Stunden bei Rückflusstemperatur gehalten. Das Lösungsmittel wurde am Rotationsverdampfer (Druck: 80 mbar; Bad: 60 °C) entfernt und der Rückstand in 1000 ml Wasser aufgenommen. Die Lösung wurde zum Entfernen von nicht umgesetztem Ethanon III drei Mal mit jeweils 300 ml Diisopropylether gewaschen. Die gewaschene wässrige Lösung wurde zwecks Reinigung mit 30 ml 5M Natronlauge und 200 ml Diisopropylether versetzt, eine Viertelstunde gerührt und die organische Phase abgetrennt. Die wässrige Phase wurde zwecks weiterer Reinigung mit 20 ml 5M Natronlauge und 200 ml Diisopropylether versetzt, wiederum eine Viertelstunde gerührt und die organische Phase abgetrennt. Die wässrige Phase wurde zwecks nochmaliger Reinigung erneut mit 20 ml 5M Natronlauge und 200 ml Diisopropylether versetzt, eine Viertelstunde gerührt und die organische Phase abgetrennt. Die verbleibende, gereinigte Wasserphase wurde mit 105 ml 50%iger Natronlauge auf pH 7,8 eingestellt und mit Diisopropylether extrahiert. Hierzu wurden 600 ml Diisopropylether zugegeben, eine Viertelstunde gerührt und die organische Phase (1. alkalischer Extrakt) abgetrennt. Die Wasserphase wurde mit weiteren 60 ml 5M Natronlauge auf pH 8,2 eingestellt und dann wieder mit Diisopropylether extrahiert. Hierzu wurden diesmal 300 ml Diisopropylether zugegeben, eine Viertelstunde gerührt und die organische Phase (2. alkalischer Extrakt) abgetrennt. Die alkalischen Extrakte 1 und 2 wurden vereinigt und mit 165 ml Wasser und 35 ml 5M Salzsäure versetzt. Es wurde eine Viertelstunde gerührt, die wässrige Phase abgetrennt, die organische Phase mit 200 ml Wasser gewaschen und am Rotationsverdampfer (Druck: bis 10 mbar; Bad: 50 °C) eingedampft. Als Eindampfrückstand wurden 473,5 g eines exo/endo-Gemischs des Propanons II im exo/endo-Verhältnis (GC) von 6,4:1 in Form eines hellbraunen Öls erhalten; das sind 54,1 % der Theorie.

### 1.2.2 Herstellung von 1-(Bicyclo[2.2.1]hept-5-en-2-yl)-3-piperidino-1-propanon (II) unter Verwendung von Piperidiniummethansulfonat

Zu 340,8 g Piperidin in 700 ml Isopropanol wurden unter Rühren und Kühlen mit Wasser innerhalb einer Stunde 392,1 g wasserfreie Methansulfonsäure getropft, wobei die Temperatur auf 75 °C anstieg. Der Tropftrichter wurde mit 50 ml Isopropanol nachgewaschen, die Mischung dann auf 25 °C abgekühlt und eine halbe Stunde bei dieser Temperatur gerührt. Das ausgefallene Produkt wurde abgesaugt, zwei Mal mit jeweils 200 ml Diisopropylether gewaschen und im Vakuum bei 50 °C getrocknet. Gewonnen wurden 688,9 g Piperidiniummethansulfonat als farblose Kristalle; das sind 95 % der Theorie.

476,7 g exo-1-(Bicyclo[2.2.1]hept-5-en-2-yl)ethanon (III-exo), 761,5 g Piperidiniummethansulfonat und 126,0 g Paraformaldehyd wurden in 950 ml Isopropanol 7 Stunden bei Rückflusstemperatur gehalten. Das Lösungsmittel wurde am Rotationsverdampfer (Druck: 80 mbar; Bad: 60 °C) entfernt und der Rückstand in 1000 ml Wasser aufgenommen. Die Lösung wurde zum Entfernen von nicht umgesetztem Ethanon III drei Mal mit jeweils 300 ml Diisopropylether gewaschen. Die gewaschene Lösung wurde zwecks Reinigung mit 30 ml 5M Natronlauge und 200 ml Diisopropylether versetzt, eine Viertelstunde gerührt und die organische Phase abgetrennt. Die wässrige Phase wurde zwecks weiterer Reinigung mit 20 ml 5M Natronlauge und 200 ml Diisopropylether versetzt, wiederum eine Viertelstunde gerührt und die organische Phase abgetrennt. Die wässrige Phase wurde zwecks nochmaliger Reinigung erneut mit 20 ml 5M Natronlauge und 200 ml Diisopropylether versetzt, eine Viertelstunde gerührt und die organische Phase abgetrennt. Die verbleibende, gereinigte Wasserphase wurde mit 90 ml 50%iger Natronlauge auf pH 7,8 eingestellt und mit Diisopropylether extrahiert. Hierzu wurden 600 ml Diisopropylether zugegeben, eine Viertelstunde gerührt und die organische Phase (1. alkalischer Extrakt) abgetrennt. Die Wasserphase wurde mit weiteren 70 ml 5M Natronlauge auf pH 8,2 eingestellt und dann wieder mit Diisopropylether extrahiert. Hierzu wurden diesmal 300 ml Diisopropylether zugegeben, eine Viertelstunde gerührt und die organische Phase (2. alkalischer Extrakt) abgetrennt. Die alkalischen Extrakte 1 und 2 wurden vereinigt und mit 165 ml Wasser und 35 ml 5M Salzsäure versetzt. Es wurde eine Viertelstunde gerührt, die wässrige Phase abgetrennt, die organische Phase mit 200 ml Wasser gewaschen und das Lösungsmittel am Rotationsverdampfer (Druck: bis 10 mbar; Bad: 50 °C) entfernt. Als Eindampfrückstand wurden 440,9 g eines exo/endo-Gemischs des Propanons II im Verhältnis (GC) von 22:1 in Form eines hellbraunen Öls erhalten; das sind 54,0 % der Theorie.

### 2. Herstellung von Biperiden (Ia)

### 2.1 Herstellung von Biperiden unter Verwendung von Diphenylmagnesium

### 2.1.1 Herstellung von Biperiden unter Verwendung von Diphenylmagnesium und 1-(Bicyclo[2.2.1]hept-5-en-2-yl)-3-piperidino-1-propanon (II) aus der Umsetzung nach 1.2.1

Zu 2000 g einer 25%igen Lösung von Phenylmagnesiumchlorid in Tetrahydrofuran wurden unter Rühren und schwachem Kühlen innerhalb einer Stunde 800 ml Dioxan gegeben. Die Temperatur stieg dabei auf 28 °C an und es bildete sich ein Niederschlag (Magnesiumchlorid-Dioxan-Komplex). Nach beendeter Dioxanzugabe wurden innerhalb einer Stunde ohne Kühlung 387,4 g eines nach Beispiel 1.2.1 erhaltenen exo/endo-Gemischs des Propanons II hinzugefügt. Dabei stieg die Temperatur auf 58 °C an. Der Tropftrichter wurde mit 30 ml Dioxan nachgewaschen, die Mischung dann auf Rückflusstemperatur erwärmt und für eine Stunde bei dieser Temperatur gehalten. Nach dem Abkühlen auf 20 °C wurde der Ansatz unter Rühren auf 800 g Eis und 600 ml Wasser gegeben. Es wurde eine Viertelstunde nachgerührt, wobei die Temperatur auf 40 °C anstieg. Die organische Phase wurde abgetrennt und die wasserhaltige Phase zwei Mal mit jeweils 500 ml Diisopropylether extrahiert. Die organischen Phasen wurden vereinigt, zwei Mal mit jeweils 500 ml Wasser gewaschen und die Lösungsmittel am Rotationsverdampfer (Druck: bis 10 mbar; Bad: 70 °C) entfernt. Der Eindampfrückstand - 532 g eines Gemischs, das im Wesentlichen aus den Enantiomerenpaaren Ia bis Id von 1-(Bicyclo[2.2.1]hept-5-en-2-yl)-1-phenyl-3-piperidino-1-propanol (I) im Verhältnis (GC) von 22,3:7,2:2,8:1 bestand (Restgehalt des Eindampfrückstands an Propanon II: 5,7 %), - wurde in 4450 ml 90%igem Isopropanol bei 60 °C gelöst und die Lösung bei dieser Temperatur mit 330 ml 5M Salzsäure versetzt. Nach der Säurezugabe wurde eine Stunde bei 60 °C und dann noch eine halbe Stunde bei Rückflusstemperatur nachgerührt. Nach dem Abkühlen auf Raumtemperatur wurden die ausgefallenen Kristalle abgetrennt und zwei Mal mit jeweils 250 ml Isopropanol gewaschen. Das so erhaltene, feuchte Hydrochlorid (320 g; entsprechend 204 g im Trockenen) wurde in 1175 ml Diisopropylether und 350 ml Wasser unter Rühren mit 130 ml 5M Natronlauge versetzt. Das Gemisch wurde auf 55 °C erwärmt und dann bei dieser Temperatur die Wasserphase abgetrennt und die Diisopropylether-Lösung zwei Mal mit jeweils 200 ml Wasser gewaschen. Aus der gewaschenen Diisopropylether-Lösung wurden bei Normaldruck 530 ml Lösungsmittel destillativ entfernt. Der Destillationsrückstand wurde abgekühlt. Nach einstündigem Rühren im Eisbad wurden die ausgefallenen Kristalle abgetrennt, mit 50 ml Diisopropylether gewaschen und im Vakuum bei 50 °C getrocknet. Erhalten wurden 139,0 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 26,9 % der Theorie.

### 2.1.2 Herstellung von Biperiden unter Verwendung von Diphenylmagnesium und 1-(Bicyclo[2.2.1]hept-5-en-2-yl)-3-piperidino-1-propanon (II) aus der Umsetzung nach 1.2.2

Zu 1000 g einer 25%igen Lösung von Phenylmagnesiumchlorid in Tetrahydrofuran wurden unter Rühren und schwachem Kühlen innerhalb einer halben Stunde 400 ml Dioxan gegeben. Die Temperatur stieg dabei auf 27 °C an und es bildete sich ein Niederschlag (Magnesiumchlorid-Dioxan-Komplex). Nach beendeter Dioxanzugabe wurden innerhalb einer Stunde ohne Kühlung 193,8 g eines nach Beispiel 1.2.2 erhaltenen exo/endo-Gemischs des Propanons II hinzugefügt. Dabei stieg die Temperatur auf 50 °C an. Der Tropftrichter wurde mit 30 ml Dioxan nachgewaschen, die Mischung dann auf Rückflusstemperatur erwärmt und für eine Stunde bei dieser Temperatur gehalten. Nach dem Abkühlen auf 15 °C wurde der Ansatz unter Rühren auf 400 g Eis und 300 ml Wasser gegeben. Es wurde eine Viertelstunde nachgerührt, wobei die Temperatur auf 38 °C anstieg. Die organische Phase wurde abgetrennt und die wasserhaltige Phase zwei Mal mit jeweils 250 ml Diisopropylether extrahiert. Die organischen Phasen wurden vereinigt, zwei Mal mit jeweils 250 ml Wasser gewaschen und die Lösungsmittel am Rotationsverdampfer (Druck: bis 10 mbar; Bad: 70 °C) entfernt. Der Eindampfrückstand - 254 g eines Gemischs, das im Wesentlichen aus den Enantiomerenpaaren Ia bis Id von 1-(Bicyclo[2.2.1]hept-5-en-2-yl)-1-phenyl-3-piperidino-1-propanol (I) im Verhältnis (GC) von 41,4:14,2:2,3:1 bestand (Restgehalt des Eindampfrückstands an Propanon II: 7,2 %), - wurde in 2225 ml 90%igem Isopropanol bei 60 °C gelöst und die Lösung bei dieser Temperatur mit 170 ml 5M Salzsäure versetzt. Nach der Säurezugabe wurde eine Stunde bei 60 °C und dann noch eine halbe Stunde bei Rückflusstemperatur nachgerührt. Nach dem Abkühlen auf Raumtemperatur wurden die ausgefallenen Kristalle abgetrennt und zwei Mal mit jeweils 100 ml Isopropanol gewaschen. Das so erhaltene, feuchte Hydrochlorid (175 g; entsprechend 102,4 g im Trockenen) wurde in 600 ml Diisopropylether und 200 ml Wasser unter Rühren mit 70 ml 5M Natronlauge versetzt. Das Gemisch wurde auf 55 °C erwärmt und dann bei dieser Temperatur die Wasserphase abgetrennt und die Diisopropylether-Lösung zwei Mal mit jeweils 100 ml Wasser gewaschen. Aus der gewaschenen Diisopropylether-Lösung wurden bei Normaldruck 300 ml Lösungsmittel destillativ entfernt. Der Destillationsrückstand wurde abgekühlt. Nach einstündigem Rühren im Eisbad wurden die ausgefallenen Kristalle abgetrennt, mit 30 ml Diisopropylether gewaschen und im Vakuum bei 50 °C getrocknet. Erhalten wurden 70,6 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 27,4 % der Theorie.

### 2.2 Herstellung von Biperiden unter Verwendung von Phenylmagnesiumbenzylalkoholat

### 2.2.1 Herstellung von Biperiden unter Verwendung von Phenylmagnesiumbenzylalkoholat und 1-(Bicyclo[2.2.1]hept-5-en-2-yl)-3-piperidino-1-propanon (II) aus der Umsetzung nach 1.2.1

Zu 2000 g einer 25%igen Lösung von Phenylmagnesiumchlorid in Tetrahydrofuran wurden unter Rühren und schwachem Kühlen innerhalb einer halben Stunde 322 g Dioxan gegeben. Die Temperatur stieg dabei auf 27 °C an und es bildete sich ein Niederschlag (Magnesiumchlorid-Dioxan-Komplex). Nach beendeter Dioxanzugabe wurden unter Kühlen bei einer Temperatur von maximal 30 °C innerhalb einer halben Stunde 197,5 g Benzylalkohol zugetropft. Anschließend wurden innerhalb einer Stunde ohne Kühlung 387,4 g eines nach Beispiel 1.2.1 erhaltenen exo/endo-Gemischs des Propanons II hinzugefügt. Dabei stieg die Temperatur auf 55 °C an. In der Folge wurde die Mischung auf Rückflusstemperatur erwärmt und für eine Stunde bei dieser Temperatur gehalten. Nach dem Abkühlen auf 20 °C wurde der Ansatz unter Rühren auf 800 g Eis und 600 ml Wasser gegeben. Es wurde eine Viertelstunde nachgerührt, die organische Phase dann abgetrennt und die wasserhaltige Phase zwei Mal mit jeweils 500 ml Diisopropylether extrahiert. Die organischen Phasen wurden vereinigt, zwei Mal mit jeweils 500 ml Wasser gewaschen und die Lösungsmittel am Rotationsverdampfer (Druck: bis 10 mbar; Bad: 70 °C) entfernt. Der Eindampfrückstand - 690 g eines Gemischs, das im Wesentlichen aus den Enantiomerenpaaren Ia bis Id von 1-(Bicyclo[2.2.1]hept-5-en-2-yl)-1-phenyl-3-piperidino-1-propanol (I) im Verhältnis (GC) von 18,3:6,0:3,2:1 bestand (Restgehalt des Eindampfrückstands an Propanon II: 1,1 %), - wurde in 4450 ml 90%igem Isopropanol bei 60 °C gelöst und die Lösung bei dieser Temperatur mit 310 ml 5M Salzsäure versetzt. Nach der Säurezugabe wurde eine Stunde bei 60 °C und dann noch eine halbe Stunde bei Rückflusstemperatur gerührt. Nach dem Abkühlen auf Raumtemperatur wurden die ausgefallenen Kristalle abgetrennt und zwei Mal mit jeweils 250 ml Isopropanol gewaschen. Das so erhaltene, feuchte Hydrochlorid (398 g; entsprechend 238,8 g im Trockenen) wurde in 1350 ml Diisopropylether und 400 ml Wasser mit 150 ml 5M Natronlauge versetzt. Das Gemisch wurde auf 55 °C erwärmt, bei dieser Temperatur die Wasserphase abgetrennt und die Diisopropylether-Lösung zwei Mal mit jeweils 200 ml Wasser gewaschen. Aus der gewaschenen Diisopropylether-Lösung wurden bei Normaldruck 600 ml Lösungsmittel destillativ entfernt. Der Destillationsrückstand wurde abgekühlt. Nach einstündigem Rühren im Eisbad wurden die ausgefallenen Kristalle abgetrennt, mit 50 ml Diisopropylether gewaschen und im Vakuum bei 50 °C getrocknet. Erhalten wurden 161,4 g Biperiden (la) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 31,2 % der Theorie.

### 2.2.2 Herstellung von Biperiden unter Verwendung von Phenylmagnesiumbenzylalkoholat und 1-(Bicyclo[2.2.1]hept-5-en-2-yl)-3-piperidino-1-propanon (II) aus der Umsetzung nach 1.2.2

Zu 2000 g einer 25%igen Lösung von Phenylmagnesiumchlorid in Tetrahydrofuran wurden unter Rühren und schwachem Kühlen innerhalb einer halben Stunde 322 g Dioxan gegeben. Die Temperatur stieg dabei auf 27 °C an und es bildete sich ein Niederschlag (Magnesiumchlorid-Dioxan-Komplex). Nach beendeter Dioxanzugabe wurden unter Kühlen bei einer Temperatur von maximal 30 °C innerhalb einer halben Stunde 197,5 g Benzylalkohol zugetropft. Anschließend wurden innerhalb einer Stunde ohne Kühlung 387,4 g eines nach Beispiel 1.2.2 erhaltenen exo/endo-Gemischs des Propanons II hinzugefügt. Dabei stieg die Temperatur auf 55 °C an. In der Folge wurde die Mischung auf Rückflusstemperatur erwärmt und für eine Stunde bei dieser Temperatur gehalten. Nach dem Abkühlen auf 20 °C wurde der Ansatz unter Rühren auf 800 g Eis und 600 ml Wasser gegeben. Es wurde eine Viertelstunde nachgerührt, die organische Phase abgetrennt und die wasserhaltige Phase zwei Mal mit jeweils 500 ml Diisopropylether extrahiert. Die organischen Phasen wurden vereinigt, zwei Mal mit jeweils 500 ml Wasser gewaschen und die Lösungsmittel am Rotationsverdampfer (Druck: bis 10 mbar; Bad: 70 °C) entfernt. Der Eindampfrückstand - 682,8 g eines Gemischs, das im Wesentlichen aus den Enantiomerenpaaren Ia bis Id von 1-(Bicyclo[2.2.1]hept-5-en-2-yl)-1-phenyl-3-piperidino-1-propanol (I) im Verhältnis (GC) von 47,0:15,1:3,6:1 bestand (Restgehalt des Eindampfrückstands an Propanon II: 0,8 %), - wurde in 4450 ml 90%igem Isopropanol bei 60 °C gelöst und die Lösung bei dieser Temperatur mit 310 ml 5M Salzsäure versetzt. Nach der Säurezugabe wurde eine Stunde bei 60 °C und dann noch eine halbe Stunde bei Rückflusstemperatur gerührt. Nach dem Abkühlen auf Raumtemperatur wurden die ausgefallenen Kristalle abgetrennt und zwei Mal mit jeweils 250 ml Isopropanol gewaschen. Das so erhaltene, feuchte Hydrochlorid (400 g; entsprechend 216,6 g im Trockenen) wurde in 1350 ml Diisopropylether und 400 ml Wasser mit 150 ml 5M Natronlauge versetzt. Das Gemisch wurde auf 55 °C erwärmt, bei dieser Temperatur die Wasserphase abgetrennt und die Diisopropylether-Lösung zwei Mal mit jeweils 200 ml Wasser gewaschen. Aus der gewaschenen Diisopropylether-Lösung wurden bei Normaldruck 600 ml Lösungsmittel destillativ entfernt. Der Destillationsrückstand wurde abgekühlt. Nach einstündigem Rühren im Eisbad wurden die ausgefallenen Kristalle abgetrennt, mit 50 ml Diisopropylether gewaschen und im Vakuum bei 50 °C getrocknet. Erhalten wurden 161,4 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 29,1 % der Theorie.

### 3. Herstellung des Biperidenhydrochlorids

93,4 g Biperiden (Ia) wurden in 1000 ml Isopropanol durch Erwärmen auf Rückflusstemperatur gelöst. Die Lösung wurde heiß filtriert und das Filter mit 100 ml Isopropanol nachgewaschen. Die vereinigten Filtrate wurden bei 75 °C mit 65 ml 5M Salzsäure versetzt. Anschließend wurde das Gemisch noch 15 Minuten zum Rückfluss erhitzt. Nach dem Abkühlen auf Raumtemperatur wurde noch eine Stunde gerührt und die ausgefallene Festsubstanz abgesaugt, zwei Mal mit jeweils 50 ml Isopropanol gewaschen und im Vakuum bei 70 °C getrocknet. Erhalten wurden 103,2 g Biperidenhydrochlorid in Form farbloser Kristalle vom Schmelzpunkt 278 bis 280 °C (Ullmanns Enzyklopädie der techn. Chemie, 4. Aufl., Band 21, Verlag Chemie, 1982, S. 627: 278 - 280 °C); das sind 98,9 % der Theorie.

## Patentansprüche

1. Verfahren zur Herstellung von Biperiden (Ia) durch Umsetzung eines exo/endo-Gemischs von 1-(Bicyclo[2.2.1]hept-5-en-2-yl)-3-piperidino-1-propanon (II) mit einem exo/endo-Verhältnis von wenigstens 4,5:1 mit einer Phenylmagnesiumverbindung ausgewaehlt zwischen Diphenylmagnesium oder Phenylmagnesiumverbindung der allgemeinen Formel 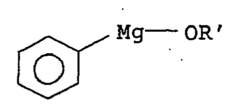 worin R' für C₁-C₄-Alkyl, wie Methyl, Ethyl, n-Propyl, iso-Propyl oder n-Butyl, C₄-C₆-Cycloalkyl, wie Cyclohexyl, C₄-C₆-Cycloalkyl-C₁-C₄-alkyl, wie 2-Cyclohexylethyl, Phenyl-C₁-C₄-alkyl, wie Benzyl, 2-Phenylethyl oder 3-Phenylpropyl, substituiertes Phenyl-C₁-C₄-alkyl, wie 3,4-(Methylendioxy)benzyl, Heteroaryl, wie 8-Chinolyl, Heteroaryl-C₁-C₄-alkyl, wie Furfuryl, 2-Thienylmethyl oder 2-(2-Thienyl)ethyl, oder Benzhydryl steht, 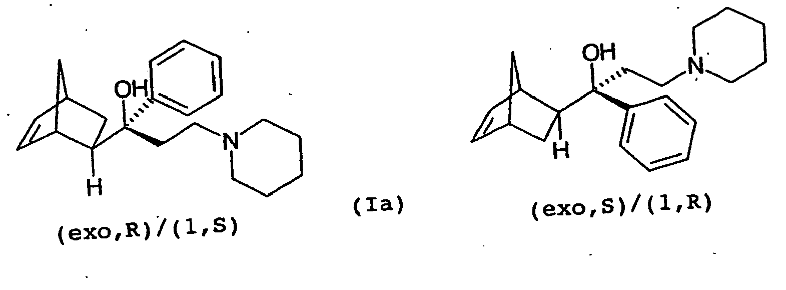 **dadurch gekennzeichnet, dass** die Herstellung des exo/endo-Gemischs von 1-(Bicyclo[2.2.1]hept-5-en-2-yl)-3-piperidino-1-propanon (II) die folgenden Schritte umfasst:
a) Umsetzung von exo-1-(Bicyclo[2.2.1]hept-5-en-2-yl)ethanon (III-exo) mit einer Formaldehydquelle und einem Säureadditionssalz von Piperidin oder mit einer Formaldehydquelle und Piperidin in Anwesenheit einer Säure in einem organischen Lösungsmittel oder in Gemischen davon mit Wasser,
b) Überführen des entstandenen Reaktionsgemischs in eine wässrige Lösung und Extraktion dieser mit einem mit Wasser begrenzt oder nicht mischbaren organischen Lösungsmittel bei einem pH-Wert von maximal 7, und
c) Extraktion des in b) erhaltenen wässrigen Raffinats, welches das exo/endo-Gemisch des 1-(Bicyclo[2.2.1]hept-5-en-2-yl)-3-piperidino-1-propanons (II) enthält, bei einem pH-Wert von wenigstens 7,5 mit einem mit Wasser begrenzt oder nicht mischbaren organischen Lösungsmittel,
d) Abtrennen des organischen Extrakts, Reinigung des organischen Extrakts über einen Auszug mit Säure und anschließendes Entfernen des Lösungsmittels, wobei man 1-(Bicyclo[2.2.1]hept-5-en-2-yl)-3-piperidino-1-propanon (II) mit einem exo/endo-Verhältnis von wenigstens 4,5:1 erhält.

2. Verfahren nach Anspruch 1, **dadurch gekennzeichnet, dass** man in Schritt a) als Säure eine organische Sulfonsäure der allgemeinen Formel RSO₃H verwendet, in der R für C₁-C₄-Alkyl, Phenyl oder C₁-C₄-Alkyl-substituiertes Phenyl steht.

3. Verfahren nach Anspruch 2, **dadurch gekennzeichnet, dass** man Methansulfonsäure einsetzt.

4. Verfahren nach Anspruch 3, **dadurch gekennzeichnet, dass** man in Schritt a) Piperidiniummethansulfonat als Säureadditionssalz von Piperidin einsetzt.

5. Verfahren nach Anspruch 1, **dadurch gekennzeichnet, dass**'man in Schritt a) Piperidin-Hydrochlorid oder Piperidin in Anwesenheit von Salzsäure oder Chlorwasserstoff einsetzt.

6. Verfahren nach einem der vorhergehenden Ansprüche, **dadurch gekennzeichnet, dass** in Schritt a) das Molverhältnis von exo-1-(Bicyclo[2.2.1]hept-5-en-2-yl)ethanon (III-exo) und Piperidin bzw. seinem Säureadditionssalz im Bereich von 1:0,9 bis 1:2 liegt.

7. Verfahren nach einem der vorhergehenden Ansprüche, **dadurch gekennzeichnet, dass** man in Schritt a) die Formaldehydquelle in einem Überschuss von 10 bis 100 Mol-% in Bezug auf das exo-1-(Bicyclo[2.2.1]hept-5-en-2-yl)ethanon (III-exo) einsetzt.

8. Verfahren nach einem der vorhergehenden Ansprüche, **dadurch gekennzeichnet, dass** man in Schritt b) nach dem Entfernen des organischen Lösungsmittels das Reaktionsgemisch aus Schritt a) in eine wässrige Lösung überführt, zuerst mit einem mit Wasser begrenzt oder nicht mischbaren organischen Lösungsmittel extrahiert, den pH-Wert des wässrigen Raffinats durch Zugabe einer Base oder einer basischen wässrigen Lösung auf einen Wert von maximal 7 einstellt, wobei die Gesamtmenge der verwendeten Base 5 bis 15 Mol-% der in der Umsetzung in Schritt a) verwendeten Menge an exo-1-(Bicyclo[2.2.1]hept-5-en-2-yl)ethanon (III-exo) beträgt, und erneut mit einem mit Wasser begrenzt oder nicht mischbaren organischen Lösungsmittel extrahiert.

9. Verfahren nach einem der vorhergehenden Ansprüche, **dadurch gekennzeichnet, dass** man in Schritt c) auf einen pH-Wert im Bereich von 8,0 bis 8,5 einstellt.

10. Verfahren nach einem der vorhergehenden Ansprüche, **dadurch gekennzeichnet, dass** man in Schritt d) zum Auszug mit Säure eine Mineralsäure verwendet.

11. Verfahren nach Anspruch 10, **dadurch gekennzeichnet, dass** man die Säure in einer Menge von 0,02 bis 0,1 Protonenäquivalenten, bezogen auf die in Schritt a) eingesetzte Menge an exo-1-(Bicyclo[2.2.1]hept-5-en-2-yl)ethanon (III-exo), einsetzt.

12. Verfahren nach einem der vorhergehenden Ansprüche, **dadurch gekennzeichnet, dass** man in Schritt a) Paraformaldehyd als Formaldehydquelle verwendet.

13. Verfahren nach einem der vorhergehenden Ansprüche, **dadurch gekennzeichnet, dass** man als Phenylmagnesiumverbindung Diphenylmagnesium oder eine Phenylmagnesiumverbindung der allgemeinen Formel 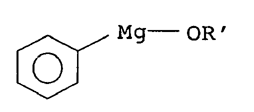 verwendet, wobei R' für C₁-C₄-Alkyl, C₄-C₆-Cycloalkyl, C₄-C₆-Cycloalkyl-C₁-C₄-alkyl, Phenyl-C₁-C₄-alkyl, substituiertes Phenyl-C₁-C₄-alkyl, Heteroaryl, Heteroaryl-C₁-C₄-alkyl oder Benzhydryl steht.

14. Verfahren nach einem der vorhergehenden Ansprüche, **dadurch gekennzeichnet, dass** die Isolierung von Biperiden (Ia) aus dem Isomerengemisch des bei der Umsetzung des exo/endo-Gemischs von 1-(Bicyclo[2.2.1]hept-5-en-2-yl)-3-piperidino-1-propanon (II) mit der Phenylmagnesiumverbindung gebildeten 1-(Bicyclo[2.2.1]hept-5-en-2-yl)-1-phenyl-3-piperidino-1-propanols (I) folgende Schritte umfasst:
- Umsetzung des Isomerengemischs mit HC1 in einem Gemisch aus Wasser und einem polaren, mit Wasser begrenzt oder vollständig mischbaren organischen Lösungsmittel und Isolierung des dabei gebildeten Hydrochlorids,
- Umsetzung des Hydrochlorids in einer Mischung aus Wasser und wenigstens einem polaren, mit Wasser begrenzt oder nicht mischbaren Dialkylether mit 4 bis 8 C-Atomen mit einer Base,
- Trennen der zwei gebildeten Phasen in der Wärme,
- Verdampfen eines Teils des Ethers aus der organischen Phase und
- Kristallisieren des Biperidens (Ia) durch Abkühlen.

## Claims

1. Process for the preparation of biperiden (Ia) 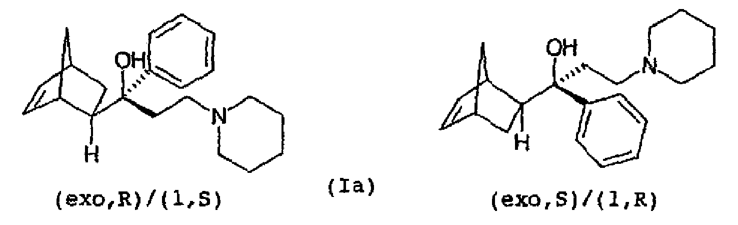 by reaction of an exo/endo mixture of 1-(bicyclo[2.2.1]hept-5-en-2-yl)-3-piperidino-1-propanone (II) having an exo/endo ratio of at least 4.5:1 with a phenylmagnesium compound selected between diphenylmagnesium or phenylmagnesium compound of the general formula 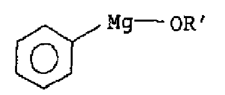 in which R' stands for C₁-C₄-alkyl, such as methyl, ethyl, n-propyl, isopropyl or n-butyl, C₄-C₆-cycloalkyl, such as cyclohexyl, C₄-C₆-cycloalkyl-C₁-C₄-alkyl, such as 2-cyclohexylethyl, phenyl-C₁-C₄-alkyl, such as benzyl, 2-phenylethyl or 3-phenylpropyl, substituted phenyl-C₁-C₄-alkyl, such as 3,4-(methylenedioxy)benzyl, heteroaryl, such as 8-quinolyl, heteroaryl-C₁-C₄-alkyl, such as furfuryl, 2-thienylmethyl or 2-(2-thienyl)ethyl, or benzhydryl, **characterized in that** the preparation of the exo/endo mixture of 1-(bicyclo[2.2.1]hept-5-en-2-yl)-3-piperidino-1-propanone (II) comprises the following steps:
a) reaction of exo-1-(bicyclo[2.2.1]hept-5-en-2-yl)ethanone (III-exo) with a formaldehyde source and an acid-addition salt of piperidine or with a formaldehyde source and piperidine in the presence of an acid in an organic solvent or in mixtures thereof with water,
b) conversion of the resultant reaction mixture into an aqueous solution and extraction thereof with an organic solvent which has limited miscibility or is immiscible with water at a pH of at most 7, and
c) extraction of the aqueous raffinate obtained in b), which comprises the exo/endo mixture of 1-(bicyclo[2.2.1]hept-5-en-2-yl)-3-piperidino-1-propanone (II), at a pH of at least 7.5 with an organic solvent which has limited miscibility or is immiscible with water,
d) separation of the organic extract, purification of the organic extract by extraction with acid and subsequent removal of the solvent, giving 1-(bicyclo-[2.2.1]hept-5-en-2-yl)-3-piperidino-1-propanone (II) having an exo/endo ratio of at least 4.5:1.

2. Process according to Claim 1, **characterized in that** the acid used in step a) is an organic sulphonic acid of the general formula RSO₃H, in which R stands for C₁-C₄₋alkyl, phenyl or C₁-C₄-alkyl-substituted phenyl.

3. Process according to Claim 2, **characterized in that** methanesulphonic acid is employed.

4. Process according to Claim 3, **characterized in that** piperidinium methanesulphonate is employed as acid-addition salt of piperidine in step a).

5. Process according to Claim 1, **characterized in that** piperidine hydrochloride or piperidine is employed in step a) in the presence of hydrochloric acid or hydrogen chloride.

6. Process according to one of the preceding claims, **characterized in that** the molar ratio of exo-1-(bicyclo[2.2.1]hept-5-en-2-yl)ethanone (III-exo) and piperidine or an acid-addition salt thereof in step a) is in the range from 1:0.9 to 1:2.

7. Process according to one of the preceding claims, **characterized in that** the formaldehyde source in step a) is employed in an excess of 10 to 100 mol% relative to the exo-1-(bicyclo[2.2.1]hept-5-en-2-yl)ethanone (III-exo).

8. Process according to one of the preceding claims, **characterized in that** after removal of the organic solvent in step b), the reaction mixture from step a) is converted into an aqueous solution, extracted firstly with an organic solvent which has limited miscibility or is immiscible with water, the pH of the aqueous raffinate is set to a value of at most 7 by addition of a base or a basic aqueous solution, where the total amount of base used is 5 to 15 mol% of the amount of exo-1-(bicyclo[2.2.1]hept-5-en-2-yl)ethanone (III-exo) used in the reaction in step a), and the mixture is re-extracted with an organic solvent which has limited miscibility or is immiscible with water.

9. Process according to one of the preceding claims, **characterized in that** a pH in the range from 8.0 to 8.5 is set in step c).

10. Process according to one of the preceding claims, **characterized in that** a mineral acid is used for the extraction with acid in step d).

11. Process according to Claim 10, **characterized in that** the acid is employed in an amount of 0.02 to 0.1 proton equivalent, based on the amount of exo-1-(bicyclo[2.2.1]hept-5-en-2-yl)ethanone (III-exo) employed in step a).

12. Process according to one of the preceding claims, **characterized in that** paraformaldehyde is used as formaldehyde source in step a).

13. Process according to one of the preceding claims, **characterized in that** the phenylmagnesium compound used is diphenylmagnesium or a phenylmagnesium compound of the general formula 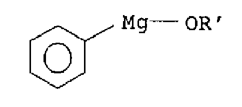 where R' stands for C₁-C₄-alkyl, C₄-C₆-cycloalkyl, C₄-C₆-cycloalkyl-C₁-C₄-alkyl, phenyl-C₁-C₄-alkyl, substituted phenyl-C₁-C₄-alkyl, heteroaryl, heteroaryl-C₁-C₄-alkyl or benzhydryl.

14. Process according to one of the preceding claims, **characterized in that** the isolation of biperiden (Ia) from the isomer mixture of the 1-(bicyclo[2.2.1]hept-5-en-2-yl)-1-phenyl-3-piperidino-1-propanol (I) formed in the reaction of the exo/endo mixture of 1-(bicyclo[2.2.1]hept-5-en-2-yl)-3-piperidino-1-propanone (II) with the phenylmagnesium compound comprises the following steps:
- reaction of the isomer mixture with HCl in a mixture of water and a polar, organic solvent which has limited miscibility or is fully miscible with water, and isolation of the hydrochloride formed in the reaction,
- reaction of the hydrochloride with a base in a mixture of water and at least one polar dialkyl ether having 4 to 8 C atoms which has limited solubility or is immiscible with water,
- separation of the two phases formed at elevated temperature,
- evaporation of some of the ether from the organic phase, and
- crystallization of the biperiden (Ia) by cooling.

## Revendications

1. Procédé de préparation du bipéridène (Ia) : 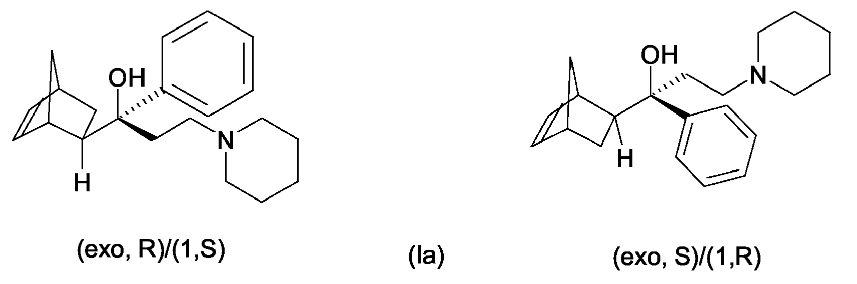 par réaction d'un mélange des isomères exo et endo de 1-(bicyclo[2.2.1]-hept-5-én-2-yl)-3-pipéridino-1-propanone (II), en un rapport exo/endo d'au moins 4,5/1, avec un composé de type phényl-magnésium, choisi parmi le diphénylmagnésium et les dérivés phénylmagnésium de formule générale 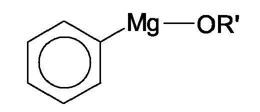 dans laquelle R' représente un groupe alkyle en C₁₋₄ tel que méthyle, éthyle, n-propyle, isopropyle ou n-butyle, cycloalkyle en C₄₋₆ tel que cyclohexyle, (cycloalkyle en C₄₋₆)-alkyle en C₁₋₄ tel que 2-cyclohexyl-éthyle, phényl-alkyle en C₁₋₄ tel que benzyle, 2-phényl-éthyle ou 3-phényl-propyle, phényl-alkyle en C₁₋₄ à substituant tel que 3,4-méthylènedioxy-benzyle, hétéroaryle tel que 8-quinolyle, hétéroaryl-alkyle en C₁₋₄ tel que furfuryle, 2-thiényl-méthyle ou 2-(2-thiényl)-éthyle, ou benzhydryle,
**caractérisé en ce que**, pour préparer le mélange d'isomères exo et endo de 1-(bicyclo[2.2.1]-hept-5-én-2-yl)-3-pipéridino-1-propanone (II), on réalise les étapes suivantes :
a) on fait réagir de l'exo-1-(bicyclo[2.2.1]-hept-5-én-2-yl)éthanone (III-exo) avec une source de formaldéhyde et un sel d'addition d'acide de pipéridine, ou avec une source de formaldéhyde et de la pipéridine en présence d'un acide, dans un solvant organique ou un mélange d'eau et d'un tel solvant ;
b) on fait du mélange réactionnel ainsi formé une solution aqueuse que l'on soumet à une extraction réalisée avec un solvant organique non-miscible ou miscible en proportion limitée à l'eau, à un pH d'au plus 7 ;
c) on soumet le raffinat aqueux obtenu dans l'étape (b), qui contient le mélange d'isomères exo et endo de 1-(bicyclo[2.2.1]-hept-5-én-2-yl)-3-pipéridino-1-propanone (II), à une extraction réalisée avec un solvant organique non-miscible ou miscible en proportion limitée à l'eau, à un pH d'au moins 7,5 ;
d) et l'on sépare l'extrait organique, on purifie cet extrait organique en le traitant avec un acide, puis on en chasse le solvant, et l'on obtient ainsi de la 1-(bicyclo[2.2.1]-hept-5-én-2-yl)-3-pipéridino-1-propanone (II) où le rapport exo/endo vaut au moins 4,5/1.

2. Procédé conforme à la revendication 1, **caractérisé en ce que** l'acide utilisé dans l'étape (a) est un acide organique sulfonique de formule générale RSO₃H où R représente un groupe alkyle en C₁₋₄, phényle, ou phényle à substituant alkyle en C₁₋₄.

3. Procédé conforme à la revendication 2, **caractérisé en ce que** l'on utilise de l'acide méthane-sulfonique.

4. Procédé conforme à la revendication 3, **caractérisé en ce que** le sel d'addition d'acide de pipéridine utilisé dans l'étape (a) est du méthane-sulfonate de pipéridinium.

5. Procédé conforme à la revendication 1, **caractérisé en ce que**, dans l'étape (a), on utilise du chlorhydrate de pipéridine, ou de la pipéridine en présence d'acide chlorhydrique ou de chlorure d'hydrogène.

6. Procédé conforme à l'une des revendications précédentes, **caractérisé en ce que**, dans l'étape (a), le rapport molaire entre l'exo-1-(bicyclo-[2.2.1]-hept-5-én-2-yl)éthanone (III-exo) et la pipéridine ou son sel d'addition d'acide vaut de 1/0,9 à 1/2.

7. Procédé conforme à l'une des revendications précédentes, **caractérisé en ce que**, dans l'étape (a), on utilise la source de formaldéhyde en un excès de 10 à 100 % en moles par rapport à l'exo-1-(bicyclo-[2.2.1]-hept-5-én-2-yl)éthanone (III-exo).

8. Procédé conforme à l'une des revendications précédentes, **caractérisé en ce que**, dans l'étape (b), on fait du mélange réactionnel issu de l'étape (a), après en avoir chassé le solvant organique, une solution aqueuse que l'on soumet d'abord à une extraction réalisée avec un solvant organique non-miscible ou miscible en proportion limitée à l'eau, on ajuste le pH du raffinat aqueux à une valeur d'au plus 7 par addition d'une base ou d'une solution aqueuse basique, la quantité de base employée représentant au total de 5 à 15 % en moles de l'exo-1-(bicyclo-[2.2.1]-hept-5-én-2-yl)éthanone (III-exo) utilisée dans la réaction de l'étape (a), et l'on opère à nouveau une extraction avec un solvant organique non-miscible ou miscible en proportion limitée à l'eau.

9. Procédé conforme à l'une des revendications précédentes, **caractérisé en ce que**, pour l'étape (c), on ajuste le pH à une valeur de 8,0 à 8,5.

10. Procédé conforme à l'une des revendications précédentes, **caractérisé en ce que**, dans l'étape (d), on emploie un acide minéral pour le traitement par un acide.

11. Procédé conforme à la revendication 10, **caractérisé en ce que** l'on emploie l'acide en une quantité représentant de 0,02 à 0,1 équivalent-proton, par rapport à la quantité d'exo-1-(bicyclo-[2.2.1]-hept-5-én-2-yl)-éthanone (III-exo) employée dans l'étape (a).

12. Procédé conforme à l'une des revendications précédentes, **caractérisé en ce que** la source de formaldéhyde utilisée dans l'étape (a) est du paraformaldéhyde.

13. Procédé conforme à l'une des revendications précédentes, **caractérisé en ce que** le composé de type phényl-magnésium utilisé est du diphénylmagnésium ou un dérivé phénylmagnésium de formule générale 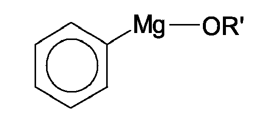 dans laquelle R' représente un groupe alkyle en C₁₋₄, cycloalkyle en C₄₋₆, (cycloalkyle en C₄₋₆)-alkyle en C₁₋₄, phényl-alkyle en C₁₋₄, phényl-alkyle en C₁₋₄ à substituant, hétéroaryle, hétéroaryl-alkyle en C₁₋₄, ou benzhydryle.

14. Procédé conforme à l'une des revendications précédentes, **caractérisé en ce que**, pour isoler le bipéridène (Ia) à partir du mélange d'isomères de 1-(bicyclo[2.2.1]-hept-5-én-2-yl)-1-phényl-3-pipéridino-1-propanol formés par réaction du mélange d'isomères exo et endo de 1-(bicyclo[2.2.1]-hept-5-én-2-yl)-3-pipéridino-1-propanone (II) avec le composé de type phényl-magnésium, on réalise les étapes suivantes :
- on fait réagir le mélange d'isomères avec du HCl, dans un mélange d'eau et d'un solvant organique polaire miscible à l'eau en toute proportion ou en proportion limitée, et l'on isole le chlorhydrate ainsi formé,
- on fait réagir ce chlorhydrate avec une base, dans un mélange d'eau et d'au moins un éther dialkylique en C₄₋₈, polaire et non-miscible ou miscible en proportion limitée à l'eau,
- on sépare à chaud les deux phases formées,
- on chasse par évaporation une partie de l'éther de la phase organique,
- et l'on fait cristalliser le bipéridène (Ia) par refroidissement.
